# EUROPÄISCHE PATENTANMELDUNG

(11) **EP 0 767 379 A1**
(43) Veröffentlichungstag der Anmeldung: **09.04.1997**
(21) Anmeldenummer: 96113180.2
(22) Anmeldetag: 15.06.1994
(51) Int. Cl.: G01N 33/574, C12P 21/08, C07K 16/28, C12Q 1/68, G01N 33/577, A61K 49/00

(54) **Verfahren zur Diagnose und Analyse von Kolonkarzinomen**

(30) Priorität: 22.06.1993 DE 4320623; 22.06.1993 DE 4320624; 02.07.1993 DE 4321944; 28.04.1994 DE 4414787
(62) Teilanmeldung aus: 94919633.1
(71) Anmelder: BOEHRINGER INGELHEIM INTERNATIONAL GmbH, D-55216 Ingelheim am Rhein (DE); Forschungszentrum Karlsruhe GmbH, 76021 Karlsruhe (DE)
(72) Erfinder: Ponta, Helmut, Prof. Dr., 76351 Linkenheim-Hochstetten (DE); Herrlich, Peter, Prof. Dr., 76229 Karlsruhe (41) (DE); Heider, Karl-Heinz, Dr., Diplom-Biologe, 1120 Wien (AT); Pals, Steven T., Dr., 1074 BE Amsterdam (NL)

(57) **Zusammenfassung**

Die Erfindung bertifft ein Verfahren zur Diagnose und Analyse von Kolonkarzinomen, das auf dem Nachweis der Expression bestimmter varianter Exons des CD44-Gens beruht. Der Nachweis kann auf Protein- oder Nukleinsäureebene erfolgen. In einer bevorzugten Ausführungsform wird die Expression in Biopsiematerial mit Hilfe exonspezifischer Antikörper nachgewiesen.

## Beschreibung

Die Erfindung betrifft Verfahren zur Diagnose und/oder Analyse von Kolonkarzinomen durch Bestimmung der Expression variabler Exons des CD44-Gens, Mittel für solche Verfahren sowie deren Verwendung.

Es besteht ein Bedarf nach verbesserten Verfahren zur Diagnose und/oder Analyse von Krebserkrankungen, insbesondere auf Grundlage von molekularen Markern.

Beispielsweise findet die hämatogene Ausbreitung von Mammakarzinomen sehr früh im Krankheitsverlauf statt und steht im Zusammenhang mit dem späteren Auftreten von Fernmetastasen (Diel *et al.,* 1992). Die molekularen Mechanismen der metastatischen Ausbreitung sind immer noch unbekannt. Die prognostischen Faktoren für die Voraussage des Metastasierungsrisikos beruhen momentan hauptsächlich auf pathologischen Kriterien, wobei die Hauptfaktoren Tumorstadium, Differenzierung (Gradierung) sowie Lymphknotenmetastasierung sind (Fisher *et al.,* 1990). Allerdings treten in Einzelfällen Diskrepanzen zwischen diesen Faktoren auf, etwa indem trotz fortgeschrittener Tumorgröße oder niedriger Differenzierung (hoher Gradierung) Lymphknotenmetastasen fehlen. Wenig untersucht ist eine Untergruppe von Patientinnen mit Lymphknoten-negativem Brustkrebs, die später Fernmetastasen entwickeln. Es besteht daher ein Bedarf an Parametern, die eine bessere Voraussage der hämatogenen Tumorausbreitung sowie einer besseren Allgemeinprognose gestatten. Ein anderes Beispiel sind Magentumoren. Sie können in zwei große histologische Kategorien eingeteilt werden, den "intestinalen" und den "diffusen" Typ (Lauren, 1965). Tumoren vom intestinalen, nicht jedoch vom diffusen Typ sind oft von einer chronischen Gastritis B und insbesondere von intestinalen Metaplasien begleitet, die für Vorläufer von dysplastischen Veränderungen und von Adenokarzinomen des intestinalen Typs gehalten werden (Jida *et* Kusama, 1982; Jass, 1983; Kato *et al.,* 1981; Sipponen *et al.,* 1983; Sirula *et al.,* 1974; Strickland *et* Mackay, 1973). Pathogenetische Unterschiede zwischen diesen beiden Adenokarzinom-Typen spiegeln sich auch in der Beobachtung wider, daß Patienten mit Tumoren vom diffusen Typ oft zur Blutgruppe A gehören, was einen möglichen Einfluß von genetischen Faktoren auf das Krebsrisiko anzeigt (Piper, 1978), während Umweltfaktoren, z.B. Infektionen mit *Helicobacter pylori,* möglicherweise für die Entwicklung von Tumoren vom intestinalen Typ wichtig sind (Parsonnet *et al.,* 1991; Nomura *et al.,* 1991). Hier wäre es wünschenswert, anhand molekularer Marker zwischen Tumoren vom intestinalen und solche vom diffusen Typ unterscheiden zu können. Als drittes Beispiel sei schließlich das Zervixkarzinom des Uterus genannt. Trotz abnehmender Inzidenz (Petterson, 1988) ist die Prognose von Patientinnen mit fortgeschrittenen Stadien von Zervixkarzinomen schlecht (Perez *et al.,* 1983; Park *et* Thigpen, 1993; Brady *et al.,* 1986). Die Frühdiagnose basiert auf der Auswertung früher morphologischer Veränderungen der Epithelzellen (zervikaler Abstrich). Auch hier ist es wünschenswert, definierte molekulare Marker zur frühen Krebserkennung, die für das Staging und als prognostische Faktoren verwendet werden können, zu finden.

Es wurde kürzlich gezeigt, daß die Expression von Varianten des Oberflächen-Glykoproteins CD44 notwendig und hinreichend ist, um sogenanntes spontanes metastatisches Verhalten sowohl in einer nicht-metastasierenden Pankreas-Adenokarzinom-Zellinie der Ratte als auch in einer nicht-metastasierenden Fibrosarkom-Zellinie der Ratte auszulösen (Günthert *et al.,* 1991). Während die kleinste CD44-Isoform, die Standardform CD44s, in einer Reihe verschiedener Gewebe, darunter Epithelzellen, ubiquitär exprimiert wird, werden bestimmte Spleißvarianten von CD44 (CD44v) nur auf einer Untergruppe von Epithelzellen exprimiert. Die CD44-Isoformen werden durch alternatives Spleißen so erzeugt, daß die Sequenzen von 10 Exons (v1-v10) in CD44s komplett ausgeschnitten werden, jedoch bei den größeren Varianten in verschiedenen Kombinationen vorkommen können (Screaton *et al.,* 1992; Heider *et al.,* 1993; Hofmann *et al.,* 1991). Die Varianten unterscheiden sich dadurch, daß an einer bestimmten Stelle des extrazellulären Teils des Proteins unterschiedliche Aminosäuresequenzen inseriert sind. Solche Varianten konnten in verschiedenen menschlichen Tumorzellen und in menschlichem Tumorgewebe nachgewiesen werden. So wurde kürzlich die Expession von CD44-Varianten im Verlauf der kolorektalen Karzinogenese untersucht (Heider *et al.,* 1993). Die Expression von CD44-Varianten fehlt in normalem menschlichem Kolonepithel, und nur eine schwache Expression ist in den proliferierenden Zellen der Krypten nachweisbar. In späteren Stadien der Tumorprogression, z.B. in Adenokarzinomen, exprimieren alle malignen Entartungen Varianten von CD44. Weiter wurde kürzlich Expression von CD44-Spleißvarianten in aktivierten Lymphozyten sowie in Non-Hodgkin-Lymphomen gezeigt (Koopman *et al.,* 1993).

Aufgabe der vorliegenden Erfindung war die Entwicklung von neuen Verfahren zur Diagnose und Analyse von Tumoren sowie die Bereitstellung von Mitteln für solche Verfahren.

Diese Aufgabe konnte mit der vorliegenden Erfindung gelöst werden. Sie betrifft ein Verfahren zur Diagnose und/oder Analyse von Tumoren, insbesondere Kolonkarzinomen, das dadurch gekennzeichnet ist, daß variantes CD44 als molekularer Marker verwendet wird.

Bevorzugt werden dabei Verfahren, die auf dem Nachweis der Expression einzelner, definierter variabler Exons des CD44-Gens oder definierter Kombinationen solcher Exons beruhen. Dabei kann es vorteilhaft sein, die Expression verschiedener varianter Exons in einer Probe zu untersuchen und zueinander ins Verhältnis zu setzen.

Besonders bevorzugt sind Verfahren, die auf dem Nachweis der Expression der variablen Exons v5 und/oder v6 beruhen.

Die erfindungsgemäßen Verfahren können an Proben außerhalb des menschlichen oder tierischen Körpers oder aber *in vivo* vorgenommen werden.

Vorteilhaft können mit den erfindungsgemäßen Verfahren Proben von Patienten untersucht werden, bei denen der Verdacht auf Kolonkarzinom besteht oder die Diagnose bereits vorliegt, der Tumor aber genauer charakterisiert werden soll. Insbesondere kann bei Verdacht auf/Diagnose von Kolonkarzinom die Expression des varianten Exons v6 untersucht werden.

Der Nachweis varianter CD44-Moleküle kann auf Proteinebene mittels Antikörpern oder auf Nukleinsäureebene mittels spezifischer Nukleinsäuresonden oder Primern für die Polymerase-Kettenreaktion (PCR) erfolgen. Die Erfindung betrifft demzufolge auch Antikörper und Nukleinsäuren, die als Sonden bzw. Primer für solche Verfahren geeignet sind, und die Verwendung solcher Antikörper und Nukleinsäuren zur Diagnose und Analyse von Tumoren, insbesondere Karzinomen.

Entsprechend betrifft die Erfindung bevorzugt auch Antikörper gegen Epitope, die von Exons v5 und/oder v6 kodiert werden, sowie Nukleinsäuren, die mit den Exons v5 und/oder v6 hybridisieren können, ferner Primer, die zur RT-PCR-Amplifikation von RNA, die die Exons v5 und/oder v6 enthält, verwendet werden können.

Zur *in-vivo*-Diagnostik können die CD44v-spezifischen Antikörper beispielsweise mit einem detektierbaren Marker, z. B. einem Radioisotop, verknüpft und in das Gefäßsystem eines Patienten injiziert werden. Nach der selektiven Bindung der Antikörper an den Tumor kann dieser mit einem geeigneten Detektionssystem visualisiert werden. Protokolle für solche immundiagnostischen Verfahren *in vivo* sind Stand der Technik (Thomas *et al.,* 1989).

Die Nuklein- und Aminosäuresequenz der varianten Exons des CD44-Gens ist bekannt (Hofmann *et al.,* 1991, Screaton *et al.,* 1992, Tölg *et al.,* 1993). Die Existenz degenerierter oder alleler Varianten ist für die Ausführung der Erfindung nicht von Bedeutung; solche Varianten sind daher ausdrücklich mit eingeschlossen.

Die Sequenz von Exon v5 des menschlichen CD44-Gens ist (SEQ ID NO: 1):

(D) V D R N G T T A Y E G N W
(CAG)AT GTA GAC AGA AAT GGC ACC ACT GCT TAT GAA GGA AAC TGG

N P E A H P P L I H H E H H E
AAC CCA GAA GCA CAC CCT CCC CTC ATT CAC CAT GAG CAT CAT GAG

E E E T P H S T S T
GAA GAA GAG ACC CCA CAT TCT ACA AGC ACA A.

Die Sequenz von Exon v6 des menschlichen CD44-Gens ist (SEQ ID NO: 3):

Q A T P S S T T E E T A T Q
TC CAG GCA ACT CCT AGT AGT ACA ACG GAA GAA ACA GCT ACC CAG

K E Q W F G N R W H E G Y R Q
AAG GAA CAG TGG TTT GGC AAC AGA TGG CAT GAG GGA TAT CGC CAA

T P R E D S H S T T G T A
ACA CCC AGA GAA GAC TCC CAT TCG ACA ACA GGG ACA GCT G.

Die erfindungsgemäßen Antikörper sind demzufolge vorzugsweise gegen Epitope innerhalb der dargestellten Aminosäuresequenzen oder deren allele Varianten gerichtet.

Ebenfalls besonders bevorzugt ist ein v6-spezifischer Antikörper, der an ein Epitop innerhalb der Aminosäuresequenz (SEQ ID NO: 10) QWFGNRWHEGYRQT oder einer allelen Variante dieser Sequenz bindet.

Besonders bevorzugt sind monoklonale Antikörper. Für das erfindungsgemäße Verfahren können jedoch auch andere Antikörpermoleküle verwendet werden, z.B. Fab- oder F(ab')₂-Fragmente von Immunglobulinen, rekombinant hergestellte single-chain-Antikörper (scFv), chimäre bzw. humanisierte Antikörper sowie andere Moleküle, die spezifisch an Epitope binden, die durch Exons v5 und/oder v6 kodiert werden. Die Herstellung von Antikörpern gegen bekannte Aminosäuresequenzen kann nach an sich bekannten Methoden erfolgen (Catty, 1989). Beispielsweise kann ein Peptid dieser Sequenz synthetisch hergestellt und als Antigen in einem Immunisierungsprotokoll eingesetzt werden. Ein anderer Weg ist die Herstellung eines Fusionsproteins, das die gewünschte Aminosäuresequenz enthält, indem eine Nukleinsäure (die synthetisch oder z.B. durch Polymerase-Kettenreaktion (PCR) aus einer geeigneten Probe hergestellt werden kann), die für diese Sequenz kodiert, in einen Expressionsvektor integriert und das Fusionsprotein in einem Wirtsorganismus exprimiert wird. Das gegebenenfalls gereinigte Fusionsprotein kann dann als Antigen in einem Immunisierungsprotokoll eingesetzt und Insert-spezifische Antikörper oder, im Falle monoklonaler Antikörper, Hybridome, die insertspezifische Antikörper exprimieren, mit geeigneten Verfahren selektiert werden. Solche Verfahren sind Stand der Technik. Heider *et al.* (1993) und Koopman *et al.* (1993) beschreiben die Herstellung von Antikörpern gegen variante Epitope von CD44.

Ebenfalls als Mittel zur Ausführung können Nukleinsäuren dienen, die mit varianten Exons, insbesondere v5 und/oder v6 hybridisieren, insbesondere solche mit einer Homologie von mehr als 80 % zu den entsprechenden Exonsequenzen. Die Herstellung solcher Nukleinsäuren kann nach an sich bekannten Methoden erfolgen. Dies gilt analog auch für Primer, die in der RT-PCR von RNA zum Nachweis der Expression der Exons v5 und/oder v6 verwendet werden können.

Die Ausführung des erfindungsgemäßen Verfahrens zur Diagnose und/oder Analyse von Tumoren, insbesondere von Karzinomen, kann zweckmäßig durch Untersuchungen von aus dem Körper entnommenen Proben, beispielsweise aus Biopsien, erfolgen. Vorteilhaft können dabei die erfindungsgemäßen Mittel verwendet werden.

Beispielsweise können Gewebsschnitte immunhistochemisch mit den erfindungsgemäßen Antikörpern mit an sich bekannten Methoden untersucht werden. Aus Gewebeproben gewonnene Extrakte oder Körperflüssigkeiten können ferner mit anderen immunologischen Methoden unter Verwendung der besagten Antikörper untersucht werden, beispielsweise in Western Blots, Enzyme-linked Immunosorbent Assays (ELISA, Catty *et* Raykundalia, 1989), Radioimmunoassays (RIA, Catty *et* Murphy, 1989) oder verwandten Immunoassays.

Der Nachweis der Expression varianter Exons kann auch auf Nukleinsäureebene erfolgen, beispielsweise durch Hybridisierung von aus Gewebeproben gewonnener RNA (Northern Blot) oder RT-PCR-amplifizierter RNA mit geeigneten Primern, oder durch Hybridisierung von Nukleinsäuren in Gewebsschnitten mit geeigneten Proben *(in-situ-*Hybridisierung).

Die Untersuchungen können qualitativ, semiquantitativ oder quantitativ erfolgen.

Durch Nachweis und/oder Quantifizierung der Expression varianter CD44-Epitope erhobene Daten können so in die Diagnose und Prognose einfließen. Vorteilhaft kann dabei die Kombination mit anderen prognostischen Parametern sein, etwa mit der Gradierung.

Die erfindungsgemäßen Verfahren und Mittel eignen sich hervorragend zur Diagnose und/oder Analyse von Tumoren, insbesondere von Kolonkarzinomen. Dies belegen die nachstehenden Beispiele.

### Abbildungen

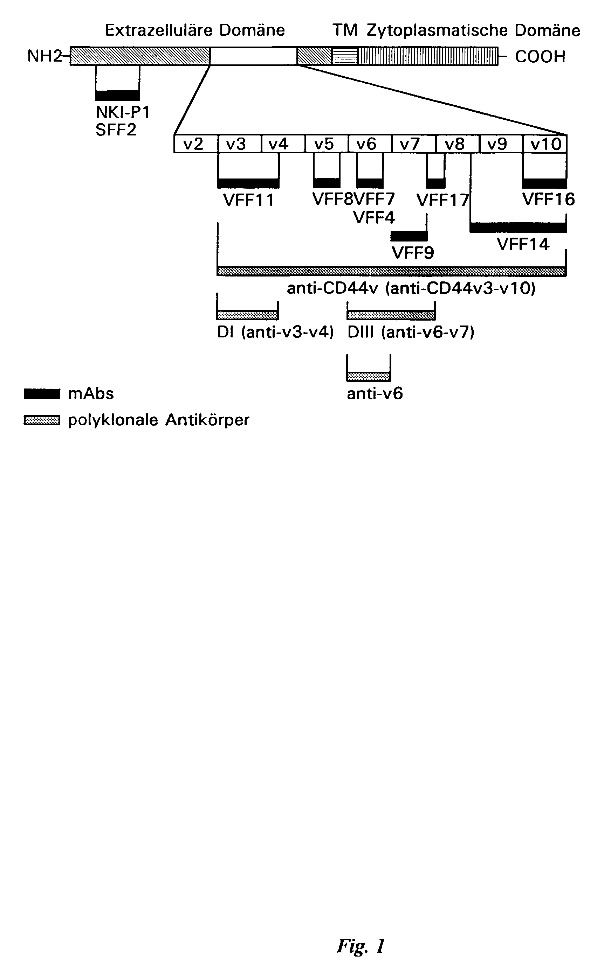

***Fig. 1:*** Schematische Darstellung eines CD44-Moleküls, das die varianten Exons v2 bis v10 enthält. Schraffierte Kästen symbolisieren CD44-Standardsequenzen (CD44s). TM = Transmembrandomäne. Die ungefähre Lokalisierung der Epitope, die von verschiedenen monoklonalen (schwarze Balken) und polyklonalen (graue Balken) Antikörpern erkannt werden, ist angezeigt. Die Antikörper VFF7, VFF8 und VFF16 sind jeweils für ein Exon spezifisch, wohingegen mAb VFF17 mit einem Epitop reagiert, daß von den Exons v7 und v8 gemeinsam kodiert wird.

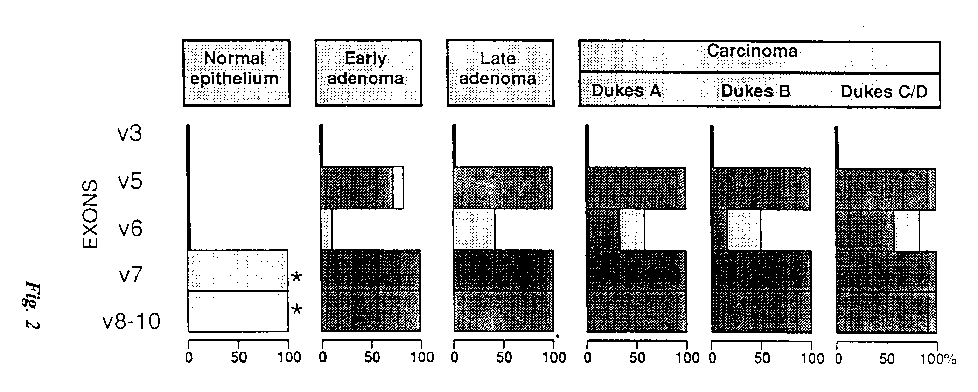

***Fig. 2:*** Expression varianter CD44-Exons in verschiedenen Stadien der kolorektalen Tumorprogression. Ergebnisse aus immunhistochemischen Anfärbungen von Gewebsschnitten.

Im folgenden wird die Eignung des erfindungsgemäßen Verfahrens zur Diagnose und/oder Analyse Kolonkarzinomen demonstriert.

### Beispiele

***Beispiel 1:Herstellung von Antikörpern gegen Epitope, die von varianten Exonsequenzen des CD44-Gens kodiert werden***

### 1.1. Klonierung von pGEX-Fusionsproteinen

Die gesamte variante Region des HPKII-Typs von CD44v (Hofmann *et al.,* 1991) wurde aus menschlicher Keratinozyten-cDNA durch Polymerase-Kettenreaktion (PCR) amplifiziert. Die beiden PCR-Primer *5*'-CAGGCTGGGAGCCAAATGAAGAAAATG-*3*' (SEQ ID NO: 11), Positionen 25-52, und *5*'-TGATAAGGAACGATTGACATTAGAGTT GGA-*3*'(SEQ ID NO: 12), Positionen 1013-984 der LCLC97-varianten Region, wie von Hofmann *et al.* beschrieben, enthielten eine *Eco*RI-Erkennungsstelle, die benutzt wurde, um das PCR-Produkt direkt in den Vektor pGEX-2T (Smith *et al.,* 1988) zu klonieren. Das resultierende Konstrukt (pGEX CD44v HPKII, v3-v10) kodiert für ein Fusionsprotein von ∼70 kD, bestehend aus Glutathion-S-transferase von *Schistosoma japonicum* und den Exons v3-v10 von humanem CD44 (Fig. 1; Heider *et al.,* 1993). Das Fusionsprotein wurde in *E*. *coli* exprimiert und anschließend über Glutathion-Agarose affinitätsgereinigt (Smith *et al.,* 1988).

Um Subklone der varianten Regionen zu erhalten, die für Affinitätsreinigungen und Western-Blot-Analysen verwendet werden konnten, wurden Fragmente kloniert, die DI (v3), DII/III (v5, v6), und DIII (v6, v7) enthielten, wobei die passenden Restriktionsschnittstellen verwendet wurden. Fusionsprotein DI enthält die CD44-Sequenz, die von Stamenkovic *et al.* (1989) beschrieben wurde, von Position 744, bis zur Position 142 der Sequenz von variantem CD44, wie sie von Hofmann *et al.* (1991) beschrieben wurde. Fusionsprotein DII/III enthält die variante Sequenz von Position 290-460, Fusionsprotein DIII die variante Sequenz von Position 378-638 (Hofmann *et al.,* 1991). Die DI und DIII enthaltenden Fragmente wurden in das pGEX-Vektorsystem, das DII/III-Fragment in den pATH-Vektor (Angel *et al.,* 1988) kloniert.

### 1.2. Monoklonale Antikörper: Immunisierung und Screening

Weibliche Balb/c Mäuse wurden intraperitoneal mit affinitätsgereinigtem Fusionsprotein (GST-CD44v3-10) immunisiert. Bei der 1. Immunisierung wurden 90 µg Fusionsprotein in komplettem Freund'schen Adiuvans, bei der 2. und 3. Immunisierung je 50 µg Fusionsprotein in inkomplettem Freund'schen Adjuvans appliziert. Die Immunisierungen erfolgten im Abstand von jeweils 4 Wochen. 14 Tage nach der letzten Immunisierung wurden die Tiere noch an drei aufeinanderfolgenden Tagen mit jeweils 10 µg Fusionsprotein in PBS immunisiert. Am darauffolgenden Tag wurden Milzzellen eines Tieres mit hohem Antikörpertiter mit P3.X63-Ag8.653-Mausmyelomzellen mit Hilfe von Polyethylenglycol 4000 fusioniert. Die Hybridomzellen wurden dann in Mikrotiterplatten in HAT-Medium selektioniert (Köhler *et* Milstein, 1975; Kearney *et al.,* 1979).

Die Bestimmung des Antikörpertiters im Serum bzw. das Screening der Hybridomüberstände wurde mit Hilfe eines ELISAs durchgeführt. Bei diesem Test werden zunächst Mikrotiterplatten mit Fusionsprotein (GST-CD44v3-10) oder nur mit Glutathion -S-transferase beschichtet. Anschließend wurde mit seriellen Verdünnungen von Serumproben bzw. Hybridomüberständen inkubiert und die spezifischen Antikörper mit Peroxidase-konjugierten Antikörpern gegen Maus-Immunglobulin nachgewiesen. Hybridome, die nur mit Glutathion-S-transferase reagierten, wurden verworfen. Die verbleibenden Antikörper wurden zunächst in einem ELISA mit domänenspezifischen Fusionsproteinen (Exon v3, Exon v5 + v6, Exon v6 + v7, Exon v8 - v10) charakterisiert (Koopman *et al.,* 1993). Ihre immunhistochemische Reaktivität wurde an menschlichen Hautschnitten getestet.

Die Exonspezifität verschiedener Antikörper ist in Fig.1 dargestellt. VFF7 erkennt ein Epitop in der Aminosäuresequenz von Exon v6. VFF8 erkennt ein Epitop in der Aminosäuresequenz von Exon v5. VFF-17 erkennt ein Epitop, das von den Exons v7 und v8 gemeinsam kodiert wird. Die Antikörper NKI-P1 und SFF2 erkennen Epitope im Standardanteil der extrazellulären Domäne in der Nähe des N-Terminus.

### 1.3. Polyklonale Antikörper

Die Herstellung und Reinigung polyklonaler Antiseren gegen die variante Region des CD44-Moleküls ist in der Literatur beschrieben (Heider *et al.,* 1993). Die Exonspezifität des Gesamtserums (anti-CD44v) sowie affinitätsgereinigter Fraktionen (anti-DI, antiDIII) ist in Fig. 1 angezeigt.

### Beispiel 2: Diagnose und Analyse von Kolonkarzinomen

### 2.1. Tumoren und Gewebe

Normale und pathologische Gewebe wurden aus den Beständen der Abteilung für Pathologie, Academic Medical Center, Universität Amsterdam, Niederlande, entnommen. Kolorektale Karzinome (n=39) wurden nach der Klassifikation von Dukes (1937, 1980) in Stadien eingeteilt, in Dukes A (n=9), Krankheit beschränkt auf die Darmwand; Dukes B (n=14), Ausdehnung über die Muskelschicht hinaus ohne Metastasierung; Dukes C/D (n=16), Tumoren mit regionalen bzw. Fernmetastasen. Adenome wurden unterteilt in frühe Adenome (Durchmesser < 1 cm, n=11) und späte Adenome (Durchmesser > 1 cm, n=12) und wurden als niedrig oder hoch differenziert nach Standardkriterien gradiert.

### 2.2. Immunhistochemie von Kolonkarzinomen

Die Herstellung der verwendeten Antikörper kann Beispiel 1 entnommen werden.

Immunfärbungen wurden nach Standardprotokollen wie früher beschrieben durchgeführt (Heider *et al.,* 1993). Gefrierschnitte (6-7 µm) wurden in eisgekühltem Methanol 4 min., dann in Aceton 1 min. fixiert, in PBS (8 g/l NaCl, 0.2 g/l KCI, 1.44 g/l Na₂HPO₄, 0.24 g/l KH₂PO₄, pH 7.4) gewaschen und mit normalem Ziegenserum (10% in PBS) präinkubiert. Dann wurden sie 3x mit PBS gewaschen und für 1 Stunde mit dem Primärantikörper (in PBS, 1% BSA) inkubiert. Für monoklonale Antikörper wurde eine Endkonzentration von 5 µg/ml verwendet. Affinitätsgereinigte polyklonale Seren wurden auf normalen Hautkeratinozyten titriert, um optimale Färbungsergebnisse zu erhalten. Endogene Peroxidase wurde mit 0.3 % H₂O₂ in Methanol blockiert und die Schnitte mit biotinyliertem Zweitantikörper (entweder anti-Maus oder anti-Kaninchen F(ab')₂, DAKO Corp., Santa Barbara, CA, USA, abhängig vom verwendeten Primärantikörper) inkubiert. Der Immunkomplex wurde mit Meerrettich-Peroxidase visualisiert, die als Streptavidin-BiotinPeroxidasekomplex an Biotin gekoppelt wurde (DAKO). Nach dreißigminütiger Inkubation mit dem Streptavidin-Biotin-Peroxidase-Komplex wurden die Schnitte mit 3,3-Amino-9-ethyl-carbazol (Sigma Chemicals, Deisenhofen, Deutschland) für 5 bis 10 min. entwickelt und die Reaktion mit H₂O abgestoppt. Die Zellen wurden mit Hämatoxylin gegengefärbt, mit Glyzerin-Gelatine eingedeckelt und mikroskopisch untersucht.

Untersucht wurde eine Gesamtzahl von 70 normalen und pathologischen Kolonproben. Tumoren wurden als "positiv" bezeichnet, wenn mehr als 10% der Tumorzellen angefärbt waren. Waren weniger als 10% der Tumorzellen gefärbt, wurde dies als "fokal" bezeichnet.

Die immunhistochemische Untersuchung von Gewebeproben ergab eine differentielle Expression von varianten CD44-Proteinen in normaler kolorektaler Mucosa, adenomatösen Polypen und kolorektalen Karzinomen (Fig. 13). Im normalen Kolonepithel ist die Expression von CD44-Proteinen begrenzt. Nur eine schwache Färbung an der Kryptenbasis zeigte sich in der Anfärbung mit einem monoklonalen Antikörper (mAb NKI-P1) gegen den N-terminalen konstanten Anteil von CD44. Ein ähnliches Expressionsmuster wurde für die Epitope beobachtet, die durch die Exons v8-v10 kodiert werden.

Expression anderer varianter Exons (v3-v6) war in normalem Kolonepithel nicht nachweisbar (Fig. 13). Wie das normale Kolonepithel, so exprimieren kolorektale Tumoren ebenfalls Spleißvarianten, die die Exons v8-v10 enthalten. Die Expression ist jedoch viel stärker und nicht mehr auf die Krypten begrenzt. Darüber hinaus werden in Tumoren zusätzliche Varianten gefunden, die im normalen Kolonepithel nicht vorhanden sind. Diese Überexpression und zunehmende Vielfalt von CD44-Varianten wird bereits in sehr frühen Stadien der kolorektalen Tumorprogression beobachtet, z.B. in frühen Adenomen, die zusätzlich zu den Exons v8-v10 zum größten Teil CD44-Isoformen exprimieren, die v5 enthalten (Fig. 13). In weiter fortgeschrittenen Stadien der kolorektalen Tumorprogression, z.B. in fortgeschrittenen Polypen und invasiven Karzinomen, nimmt das Ausmaß der Expression von v5-enthaltenden CD44-Varianten zu. Überraschend war jedoch, daß die Tumorprogression stark mit der Expression v6 enthaltender CD44-Isoformen korrelierte (Fig. 13): Expression dieses Exons war nachweisbar in keiner der normalen Kolonproben, in 9% der frühen Polypen, in 45% der fortgeschrittenen Polypen und in 67% der invasiven Karzinome. (Chi-square 1 df p < 0.0001). Darüber hinaus war die Expression von v6 in Karzinomen signifikant korreliert zum Dukes-Stadium. Während der Anteil positiver Proben in den nicht-metastatischen Dukes A und B Tumoren 52% betrug, war er 83% in der metastatischen Dukes C/D-Gruppe (Chi-square 1 df p < 0.05). Zusätzlich konnte beobachtet werden, daß die Überexpression v6 enthaltender CD44-Isoformen während der Tumorprogression nicht nur durch die steigende Zahl positiver Fälle reflektiert wird, sondern auch durch eine zunehmende Zahl positiver Zellen innerhalb eines Tumor sowie durch ein höheres Expressionsniveau (stärkere Anfärbung der Zellen). Die fokale Expression von v6 in Adenomen war mit einem weiteren Parameter der Tumorprogression korreliert, dem histologischen Tumorgrad, wobei in keinem von 17 Adenomen mit niedrigem, jedoch 5 von 6 Adenomen mit hohem Grad v6-Expression positiv war.

Informationen über die v6-Expression, wie sie z.B. durch routinemäßige Immunhistochemie erhalten werden können, können also wegen des überraschenden Zusammenhangs speziell der v6-Expression mit dem Tumorstadium wertvolle Erkenntnisse für Diagnose und Prognose von Kolonkarzinomen ermöglichen.

### Literatur

Allen, R.W., Trach, K.A., and Hoch, J.A. Identification of the 37kDa protein displaying a variable interaction with the erythroid cell membrane as glyceraldehyde-3-phosphate dehydrogenase. *J Biol. Chem. 262:* 649-652 (1987).

Angel P., Allegretto, E.A., Okuio, S.T.Hatton, K., Boyle, W.J., Hunter, T., Karin, M. Oncogene *jun* encodes a sequence-specific trans-activator similar to AP-1. *Nature 332*: 166 (1988).

Arch, R., Wirth, K., Hofmann, M., Ponta, H., Matzku, S., Herrlich, P., and Zöller, M. Participation in normal immune responses of a splice variant of CD44 that encodes a metastasis-inducing domain. *Science 257:* 682-685 (1992).

Brady, L.W., Perez, C.A., Bedwinnek, J.M. Failure patterns in gynecologic cancer. *Int. J. Rad. Oncol. Biol. Phys. 12:* 549-557 (1986).

Catty, D (Hrsg). *Antibodies Vols. I und II*. IRL Press Oxford (1989).

Catty, D., Raykundalia, C. ELISA and related immunoassays. In: Catty, D (Hrsg). *Antibodies Vol. II.* IRL Press Oxford (1989), 97-152, s. S. 105-109.

Catty, D., Murphy, G. Immunoassays using radiolabels. In: Catty, D (Hrsg). *Antibodies Vol. II.* IRL Press Oxford (1989), 77-96.

Diehl, I. J., Kaufmann, M., Goerner R., Costa, S. D., Kaul, S., Bastert, G. Detection of tumor cells in bone marrow of patients with primary breast cancer: A prognostic factor for distant metastasis. *J. Clin. Oncol. 10:* 1534-1539 (1992).

Fisher, E. R., Redmond, C., Fisher, B., Bass, G. Pathologic findings from the National Surgical Adjuvant Breast and Bowel Projects (BSABP). Prognostic discriminants for 8-year survival for node-negative invasive breast cancer patients. *Cancer 65:* 2121-2128 (1990).

Günthert, U., Hofmann, M., Rudy, W., Reber, S., Zöller, M., Haußmann, I., Matzku, S., Wenzel, A., Ponta, H., and Herrlich, P. A new variant ofglycoprotein CD44 confers metastatic potential to rat carcinoma cells. *Cell 65:* 13-24 (1991).

Heider, K.-H., Hofmann, M., Horst, E., van den Berg, F., Ponta, H., Herrlich, P., and Pals, S.T.A human homologue ofthe rat metastasis-associated variant ofCD44 is expressed in colorectal carcinomas and adenomatous polyps. *J*. *Cell Biol. 120:* 227-233 (1993).

Hofmann, M., Rudy, W., Zöller, M., Tölg, C., Ponta, H., Herrlich P., and Günthert, U. CD44 splice variants confer metastatic behavior in rats: homologous sequences are expressed in human tumor cell lines. *Cancer Res. 51:* 5292-5297 (1991).

Jass, J.R.A classification of gastric dysplasia. *Histopath. 7:* 181-193 (1983).

Jida, F., and Kusama, J. Gastric and intestinal metaplasia. Significance of type of intestinal metaplasia upon development of gastric carcinoma. *Cancer 50:* 2854-2858 (1982).

Kato, Y., Kiagawa T., Nakamura, K., and Sugano, H. Changes in the histologic tpyes of gastric carcinoma in Japan. *Cancer 48:* 2084-2087 (1981).

Kearney, J.F., Radbruch A., Liesegang B., Rajewski K. A new mouse myeloma cell line that has lost imunoglobulin expression but permits construction ofantibody-secreting hybrid cell lines. *J. Immunol. 123:* 1548 (1979).

Köhler, G., Milstein, C. Continous culture of fused cells secreting antibody of predefined specifity. *Nature 265:* 495 (1975)

Koopman, G., Heider, K.-H., Horts, E., Adolf, G. R., van den Berg, F., Ponta, H., Herrlich, P., Pals, S. T. Activated human lymphocytes and aggressive Non-Hodgkin's lymphomas express a homologue ofthe rat metastasis-associated variant ofCD44. *J. Exp. Med. 177:* 897-904 (1993).

Laemmli, U. Cleavage of structural proteins during the assembly ofthe head of bacterio-phage T4. *Nature 227:* 680-685 (1970).

Lauren P. The two histological main types of gastric carcinoma: diffuse and so-called intestinal-type carcinoma. *Acta Path. Microbiol. Scand 64:* 31-49 (1965).

Mackay, C. R., Terpe, H.-J., Stauder, R., Marston, W. L., Stark, H., Günthert U. Expression and modulation ofCD44 variant isoforms in humans. *J. Cell Biol. 124:* 71-82 (1994).

Mayer, B., Jauch, K.W., Günthert, U., Figdor, C.G., Schildberg, F.W., Funke, I., Johnson, J.P. De-novo expression ofCD44 and survival in gastric cancer. *Lancet 342:* 1019-1022 (1993).

Nomura, A., Stemmermann, G.N., Chyon, P.H., Kato, I., Perez-Perez, G.I. and Blaser, H.J. *Helicobacter pylori* infection and gastric carcinoma among Japanese americans in Hawaii. *New Engl. J. Med. 325*: 1132-1136 (1991).

Park, R.C., Thigpen, J.T. Chemotherapy in advanced and recurrent cervical cancer. *Cancer 71*: 1446-1450 (1993).

Parsonnet, J., Friedman, G.D., Vandersteen, D.F., Chang, Y., Vogelman, J.H., Orentreich, N., and Sikley, R.K. Helicobacter pylori infection and the risk of gastric carcinoma. *New Engl. J. Med. 325:* 1127-1131 (1991).

Perez, C.A., Breaux, S., Madoo-Jones, H., Bedwinek, J.M., Camel, H.M., Purdy, J.A., Walz, B.J. Radiation therapy alone in the treatment ofcarcinoma ofuterine Zervix. *Cancer 51:* 1393-1402 (1983).

Petterson, F. Annual report on results oftreatment in gynecologic cancer. *FIGO Cancer Comitees: Vol. 20* (1988).

Piper, D.W. Stomach cancer. In: "Geneva: International Union Against Cancer". *UICC Technical Report Series Vol. 34* (1978).

Sambrook, J., Fritsch E.E., Maniatis I., *Molecular cloning.* Cold Spring Harbor Laboratory Press, Cold Spring Harbor (1989).

Screaton, G.R., Bell, M.V., Jackson, D.G., Cornelis, F.B., Gerth, U., and Bell, J. I. Genomic structure of DNA encoding the lymphocyte homing receptor CD44 reveals at least 12 alternatively spliced exons. *Proc. Natl. Acad. Sci. U.S.A. 89:* 12160-12164 (1992).

Sipponen, P., Kekki, M., and Surala, M. Atrophic chronic gastritis and intestinal metaplasia in gastric carcinoma. Comparison with a representative population sample. *Cancer 52:* 1062-1068 (1983).

Siurala, M., Lehtola, J., and Ihamäki, T. Atrophic gastritis and its sequelae. Results of 15-23 years follow-up. *Scand. J. Gastroenterol. 1:* 40-48 (1974).

Smith, D.B., Johnson, K.S. Single-step purification of polypetides expressed in *Escherichia coli* as fusions with glutathione S-transferase. *Gene 67:* 31 (1988).

Stamenkovic, I., Amiot, M., Pesando, J.M., and Seed, B.A lymphocyte molecule implicated in lymph node homing is a member ofthe cartilage link protein family. *Cell 56:* 1057-1062 (1989).

Strickland, R.G., and Mackay, I.R.A reappraisal ofthe nature and significance ofchronic atrophic gastritis. *Dig. Dis. Sci. 18:* 426-440 (1973).

Tölg, C., Hofmann, M., Herrlich, P., and Ponta, H. Splicing choice from ten variant exons establishes CD44 variability. *Nucleic Acids. Res. 21:* 1225-1229 (1993).

Thomas, G. D., Dykes, P. W., Bradwell, A. R. Antibodies for tumour immunodetection and methods for antibody radiolabeling. In: Catty, D. (Hrsg.). *Antibodie Vol. II.* IRL Press Oxford, 223-244 (1989).

## Patentansprüche

1. Verfahren zur Diagnose und/oder Analyse von Kolonkarzinomen *in vitro,* dadurch gekennzeichnet, daß es auf dem Nachweis der Expression des varianten Exons v6 des CD44-Gens beruht.

2. Verfahren nach Anspruch 1, dadurch gekennzeichnet, daß der Nachweis ein immunologischer Nachweis ist.

3. Verfahren nach Anspruch 1, dadurch gekennzeichnet, daß es auf dem Nachweis einer Nukleinsäure beruht, die spezifisch für Exon v6 ist.

4. Verwendung eines Antikörpers, der gegen ein Epitop gerichtet ist, das von dem varianten Exon v6 des CD44-Gens kodiert wird, in einem Verfahren gemäß einem der Ansprüche 1 oder 2.

5. Verwendung eines Antikörpers, der gegen ein Epitop gerichtet ist, das von dem varianten Exon v6 des CD44-Gens kodiert wird, zur Herstellung eines Mittels für die Diagnose und/oder Analyse von Kolonkarzinomen.

6. Verwendung einer Nukleinsäure in einem Verfahren gemäß Anspruch 3.


SEQUENZPROTOKOLL

(1) ALLGEMEINE ANGABEN:

(i) ANMELDER:
(A) NAME: Boehringer Ingelheim International GmbH
(B) STRASSE: Binger Strasse
(C) ORT: Ingelheim
(E) LAND: Deutschland
(F) POSTLEITZAHL: 55218
(G) TELEFON: 49-6132-772770
(H) TELEFAX: 49-6132-4473

(A) NAME: Forschungszentrum Karlsruhe GmbH
(B) STRASSE: Postfach 3640
(C) ORT: Karlsruhe
(E) LAND: Deutschland
(F) POSTLEITZAHL: 76021

(ii) BEZEICHNUNG DER ERFINDUNG: Verfahren zur Diagnose und Analyse von
Kolonkarzinomen

(iii) ANZAHL DER SEQUENZEN: 14

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 120 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..119

(ix) MERKMAL:
(A) NAME/SCHLÜSSEL: exon
(B) LAGE:1..120

(xi) SEQUENZBESCHREIBUNG: SEQ ID NO: 1:

CA GAT GTA GAC AGA AAT GGC ACC ACT GCT TAT GAA GGA AAC TGG AAC 47
Asp Val Asp Arg Asn Gly Thr Thr Ala Tyr Glu Gly Asn Trp Asn
1               5                   10                  15

CCA GAA GCA CAC CCT CCC CTC ATT CAC CAT GAG CAT CAT GAG GAA GAA 95
Pro Glu Ala His Pro Pro Leu Ile His His Glu His His Glu Glu Glu
                20                  25                  30

GAG ACC CCA CAT TCT ACA AGC ACA A 120
Glu Thr Pro His Ser Thr Ser Thr

            35

(2) ANGABEN ZU SEQ ID NO: 2:

(i) SEQUENZKENNZEICHEN:
(A) LÄNGE: 39 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

Asp Val Asp Arg Asn Gly Thr Thr Ala Tyr Glu Gly Asn Trp Asn Pro
1               5                   10                  15

Glu Ala His Pro Pro Leu Ile His His Glu His His Glu Glu Glu Glu
            20                  25                  30

Thr Pro His Ser Thr Ser Thr
        35

(2) ANGABEN ZU SEQ ID NO: 3:

(i) SEQUENZKENNZEICHEN:
(A) LÄNGE: 129 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..128

(ix) MERKMAL:
(A) NAME/SCHLÜSSEL: exon
(B) LAGE:1..129

(xi) SEQUENZBESCHREIBUNG: SEQ ID NO: 3:

TC CAG GCA ACT CCT AGT AGT ACA ACG GAA GAA ACA GCT ACC CAG AAG          47
   Gln Ala Thr Pro Ser Ser Thr Thr Glu Glu Thr Ala Thr Gln Lys
    40                  45                  50

GAA CAG TGG TTT GGC AAC AGA TGG CAT GAG GGA TAT CGC CAA ACA CCC          95
Glu Gln Trp Phe Gly Asn Arg Trp His Glu Gly Tyr Arg Gln Thr Pro
 55                  60                  65                  70

AGA GAA GAC TCC CAT TCG ACA ACA GGG ACA GCT G                           129
Arg Glu Asp Ser His Ser Thr Thr Gly Thr Ala
                75                  80

(2) ANGABEN ZU SEQ ID NO: 4:

(i) SEQUENZKENNZEICHEN:
(A) LÄNGE: 42 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

Gln Ala Thr Pro Ser Ser Thr Thr Glu Glu Thr Ala Thr Gln Lys Glu
1               5                   10                  15

Gln Trp Phe Gly Asn Arg Trp His Glu Gly Tyr Arg Gln Thr Pro Arg
            20                  25                  30

Glu Asp Ser His Ser Thr Thr Gly Thr Ala
        35                  40

(2) ANGABEN ZU SEQ ID NO: 5:

(i) SEQUENZKENNZEICHEN:
(A) LÄNGE: 132 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..131

(ix) MERKMAL:
(A) NAME/SCHLÜSSEL: exon
(B) LAGE:1..132

(xi) SEQUENZBESCHREIBUNG: SEQ ID NO: 5:

CA GCC TCA GCT CAT ACC AGC CAT CCA ATG CAA GGA AGG ACA ACA CCA      47
   Ala Ser Ala His Thr Ser His Pro Met Gln Gly Arg Thr Thr Pro
           45                  50                  55

AGC CCA GAG GAC AGT TCC TGG ACT GAT TTC TTC AAC CCA ATC TCA CAC      95
Ser Pro Glu Asp Ser Ser Trp Thr Asp Phe Phe Asn Pro Ile Ser His
        60                  65                  70

CCC ATG GGA CGA GGT CAT CAA GCA GGA AGA AGG ATG G                   132
Pro Met Gly Arg Gly His Gln Ala Gly Arg Arg Met
    75                  80                  85

(2) ANGABEN ZU SEQ ID NO: 6:

(i) SEQUENZKENNZEICHEN:
(A) LÄNGE: 43 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 6:

Ala Ser Ala His Thr Ser His Pro Met Gln Gly Arg Thr Thr Pro Ser
1               5                   10                  15

Pro Glu Asp Ser Ser Trp Thr Asp Phe Phe Asn Pro Ile Ser His Pro
            20                  25                  30

Met Gly Arg Gly His Gln Ala Gly Arg Arg Met

35                    40

(2) ANGABEN ZU SEQ ID NO: 7:

(i) SEQUENZKENNZEICHEN:
(A) LÄNGE: 102 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: CDS
(B) LAGE:3..101

(ix) MERKMAL:
(A) NAME/SCHLÜSSEL: exon
(B) LAGE:1..102

(xi) SEQUENZBESCHREIBUNG: SEQ ID NO: 7:

AT ATG GAC TCC AGT CAT AGT ACA ACG CTT CAG CCT ACT GCA AAT CCA          47
   Met Asp Ser Ser His Ser Thr Thr Leu Gln Pro Thr Ala Asn Pro
        45                  50                  55

AAC ACA GGT TTG GTG GAA GAT TTG GAC AGG ACA GGA CCT CTT TCA ATG          95
Asn Thr Gly Leu Val Glu Asp Leu Asp Arg Thr Gly Pro Leu Ser Met
     60                  65                  70

ACA ACG C                                                                102
Thr Thr
 75

(2) ANGABEN ZU SEQ ID NO: 8:

(i) SEQUENZKENNZEICHEN:
(A) LÄNGE: 33 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 8:

Met Asp Ser Ser His Ser Thr Thr Leu Gln Pro Thr Ala Asn Pro Asn
  1               5                  10                  15

Thr Gly Leu Val Glu Asp Leu Asp Arg Thr Gly Pro Leu Ser Met Thr
             20                  25                  30

Thr

(2) ANGABEN ZU SEQ ID NO: 9:

(i) SEQUENZKENNZEICHEN:
(A) LÄNGE: 78 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 9:

Ala Ser Ala His Thr Ser His Pro Met Gln Gly Arg Thr Thr Pro Ser
1               5                   10                  15

Pro Glu Asp Ser Ser Trp Thr Asp Phe Phe Asn Pro Ile Ser His Pro
            20                  25                  30

Met Gly Arg Gly His Gln Ala Gly Arg Arg Met Asp Met Asp Ser Ser
        35                  40                  45

His Ser Thr Thr Leu Gln Pro Thr Ala Asn Pro Asn Thr Gly Leu Val
    50                  55                  60

Glu Asp Leu Asp Arg Thr Gly Pro Leu Ser Met Thr Thr Gln
65                  70                  75

(2) ANGABEN ZU SEQ ID NO: 10:

(i) SEQUENZKENNZEICHEN:
(A) LÄNGE: 14 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 10:

Gln Trp Phe Gly Asn Arg Trp His Glu Gly Tyr Arg Gln Thr
1               5                   10

(2) ANGABEN ZU SEQ ID NO: 11:

(i) SEQUENZKENNZEICHEN:
(A) LÄNGE: 27 Basenpaare
(B) ART: Nucleotid
(C) STRANGFORM: Einzelstrang
(D) TOPOLOGIE: linear

(ii) ART DES MOLEKÜLS: cDNA zu mRNA

(xi) SEQUENZBESCHREIBUNG: SEQ ID NO: 11:

CAGGCTGGGA GCCAAATGAA GAAAATG                                    27

(2) ANGABEN ZU SEQ ID NO: 12:

(i) SEQUENZKENNZEICHEN:
(A) LÄNGE: 30 Basenpaare
(B) ART: Nucleotid
(C) STRANGFORM: Einzelstrang
(D) TOPOLOGIE: linear

(ii) ART DES MOLEKÜLS: cDNA zu mRNA

(xi) SEQUENZBESCHREIBUNG: SEQ ID NO: 12:

TGATAAGGAA CGATTGACAT TAGAGTTGGA 30

(2) ANGABEN ZU SEQ ID NO: 13:

(i) SEQUENZKENNZEICHEN:
(A) LÄNGE: 39 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 13:

Asp Val Asp Arg Asn Gly Thr Thr Ala Tyr Glu Gly Asn Trp Asn Pro
1               5                   10                  15

Glu Ala His Pro Pro Leu Ile His His Glu His His Glu Glu Glu Glu
            20                  25                  30

Thr Pro His Ser Thr Ser Thr
        35

(2) ANGABEN ZU SEQ ID NO: 14:

(i) SEQUENZKENNZEICHEN:
(A) LÄNGE: 42 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 14:

Gln Ala Thr Pro Ser Ser Thr Thr Glu Glu Thr Ala Thr Gln Lys Glu
1               5                   10                  15

Gln Trp Phe Gly Asn Arg Trp His Glu Gly Tyr Arg Gln Thr Pro Arg
            20                  25                  30

Glu Asp Ser His Ser Thr Thr Gly Thr Ala
        35                  40